# EUROPEAN PATENT SPECIFICATION

(11) **EP 0 652 196 B1**
(45) Date of publication and mention of the grant of the patent: **17.05.2000**
(21) Application number: 94116947.6
(22) Date of filing: 26.10.1994
(51) Int. Cl.: C07C 17/10, C07C 19/01

(54) **Process for the preparation of highly chlorinated paraffins**
Verfahren zur Herstellung von hochchlorierten Parraffinen
Procédé pour la préparation de paraffines hautement chlorées

(30) Priority: 09.11.1993 DE 4338195
(43) Date of publication of application: 10.05.1995
(73) Proprietor: DOVER CHEMICAL LTD., Douglas, Isle of Man (GB)
(72) Inventor: Bewart, Dietmar, Dr., D-86368 Gersthofen (DE); Freyer, Walter, Dr., D-86391 Stadtbergen (DE)
(74) Representative: Senior, Janet

(56) References cited:
- DE-A- 1 443 892
- DE-A- 1 905 923

## Description

Bei der Herstellung von chlorierten Kohlenwasserstoffen, die 14 bis 40 Kohlenstoffatome aufweisen und einen Chlorgehalt von mehr als 60% Chlor aufweisen, wird die Chlorierung in einem Lösemittel durchgeführt, da die Schmelzsviskosität der gebildeten chlorierten Paraffine sehr hoch ist und der Chloraustausch zwischen der gasförmigen und der flüssigen Phase nicht mehr normal stattfindet. Als Lösemittel wird vornehmlich Tetrachlorkohlenstoff eingesetzt, neuerdings auch Chloroform. Auch die Verwendung von Chlorfluorkohlenwasserstoffen ist in der Literatur beschrieben (vgl. DE 2150599). DE 1443892 beschreibt ein Verfahren zur Herstellung von hochchlorierten Kohlenwasserstoffen, bei welchem statt eines chlorierten Lösungsmittels überschüssiges Chlor unter Druck als Lösungsmittel eingesetzt wird. DE 1905923 offenbart ein Verfahren zur Monochlorierung von C₁₄-C₁₈ Paraffinen. Die Umsetzung zu den Chlorparaffinen erfolgt unter Verwendung von verflüssigtem Chlor und in Gegenwart von einem freie Radikale bildenden Katalysator, wie beispielsweise organischen Peroxiden, oder durch Einwirkung von ultravioletten Strahlen, Röntgenstrahlen, etc..

Da Chlorkohlenwasserstoffe wegen ihrer die Gesundheit und die Umwelt schädigenden Eigenschaften besonderen Aufwand bei ihrer Handhabung erfordern, bestand die Aufgabe, ihre Menge im Produktionskreislauf so weit wie möglich zu verringern.

Es wurde gefunden, daß die Herstellung hochchlorierter Paraffine auch in Abwesenheit eines Lösemittels möglich ist.

Die Erfindung stellt daher ein Verfahren zur Herstellung eines chlorierten C₁₄-C₄₀ Paraffins mit einem Chlorgehalt von mehr als 60 Gew.-% aus einem C₁₄-C₄₀ Paraffinreaktanten, der ein gesättigter oder ungesättigter Kohlenwasserstoff ist, welcher bereits Chloratome enthalten kann, zur Verfügung, wobei das Verfahren umfaßt:

Umsetzen des C₁₄-C₄₀ Paraffinreaktanten mit einer Menge an flüssigem Chlor, die vollständig mit dem Paraffin reagiert und eine chlorierte Paraffinphase des chlorierten C₁₄-C₄₀ Paraffins mit einem Chlorgehalt von mehr als 60 Gew.-% bildet, unter einem Druck von bis zu 10 bar in Abwesenheit eines organischen Lösungsmittels bei einer erhöhten Temperatur im Bereich von 75 °C bis zu 140°C in Gegenwart einer Quelle für freie Radikale, wobei das C₁₄-C₄₀ Paraffin im innigen Gemisch mit einer wäßrigen Phase vorliegt, das innige Gemisch durch Rühren aufrechterhalten wird und die Reaktion fortgesetzt wird, bis eine chlorierte Paraffinphase und eine wäßrige Salzsäurephase gebildet worden ist, und

Trennen der chlorierten Paraffinphase von der wäßrigen Phase, worin der Trennschritt dadurch durchgeführt wird, daß das Rühren beendet wird.

Als Ausgangsprodukt für das erfindungsgemäße Verfahren dienen gesättigte oder ungesättigte Kohlenwasserstoffe mit 14 bis 40, vorzugsweise 17 bis 24 C-Atomen, die bereits Chloratome enthalten können ("anchloriert"). Zu diesen Produkten gehören die verschiedenen Paraffinsorten, beispielsweise n-Paraffine, α-Olefine.

Die Chlorierung wird mittels flüssigem Chlor durchgeführt. Der Druck im Reaktionsgefäß beträgt demgemäß bis zu 10 bar, vorzugsweise 2 bis 6 bar. Die Reaktionstemperatur beträgt 75 bis 140, vorzugsweise 80 bis 105°C.

Die Reaktion wird in Gegenwart eines freie Radikale bildenden Katalysators durchgeführt. Die Bindungen der -COOC-, -COOOOC-, bzw. -CNNC-Verbindungen dissoziieren bereits bei niederen Temperaturen und bilden freie Radikale.

Vorzugsweise besteht der freie Radikale bildende Katalysator aus einer Azoverbindung, beispielsweise einem Azonitril wie α,α-Azo-di-isobutyronitril, oder einem organischen Peroxid wie Benzoylperoxid oder Dilauroylperoxid oder einem organischen Hydroperoxid. Bevorzugt werden α,α-Azo-di-isobutyronitril und Dilauroylperoxid verwendet.

Es ist jedoch auch möglich, zur Bildung der freien Radikale ultraviolettes Licht oder andere ionisierende Strahlung, wie beispielsweise Röntgenlicht, anzuwenden.

Die Umsetzung der Paraffine mit dem Chlor erfolgt in Gegenwart von Wasser, welches den bei der Reaktion entstehenden Chlorwasserstoff absorbiert, oder verdünnter Salzsäure.

Das erfindungsgemäße Verfahren kann in verschiedenen Apparaturen durchgeführt werden. Möglich sind Rührkessel, Fallfilmreaktoren, Schlaufenreaktoren, Blasensäulenreaktoren. Wesentlich ist, daß eine intensive Durchmischung der wässrigen und der organischen Phase stattfindet. Vorzugsweise wird ein Rührkessel mit Impellerrührer verwendet.

Die Erfindung stellt auch ein Verfahren zur Herstellung von hochchlorierten Paraffinen mit einem Chlorgehalt von mehr als 60 Gew.-% bei einer Temperatur von 75 bis 140 °C und einem Druck von bis zu 10 bar durch Umsetzen eines C₁₄-C₄₀ Paraffins, das ein gesättigter oder ungesättigter Kohlenwasserstoff ist, welcher bereits Chloratome enthalten kann, mit flüssigem Chlor, zur Verfügung, worin
das Chlor vollständig reagiert, die Reaktion in Gegenwart einer derartigen Wassermenge durchgeführt wird, daß während der Chlorierung Salzsäure mit einer Stärke von 29 % (m/m) bis 35 % (m/m) gebildet wird, in Gegenwart einer Quelle für freie Radikale und in Abwesenheit eines organischen Lösungsmittels.

Durch das erfindungsgemäße Verfahren ist es möglich, hochchlorierte Paraffine in einfacher Weise herzustellen. Es hat sich gezeigt, daß die Anwesenheit von Salzsäure auch bei den hohen Temperaturen keine Qualitätseinbuße bewirkt. Die Trennung des Chlorparaffins von der Salzsäure ist unter diesen Bedingungen sehr gut. Eine den Transport des Chlorparaffins in nachgeschaltete Apparate behindernde Emulsionsschicht ("Mulmschicht") tritt nicht auf.

Die nachfolgenden Beispiele sollen die Erfindung näher erläutern.

Die Versuche wurden in einem 8 m³ großen stahlemaillierten Rührwerkskessel durchgeführt, der mit einem Impellerrührer ausgerüstet wurde. Das Reaktionsgemisch wurde mit dem 3 Flügel aufweisenden Rührer in Verbindung mit Prallflächen, sogenannten Stromstören, gerührt. Der Chlorierer war mit Leitungen bestückt, mit Hilfe derer die Rohstoffe wie Paraffin, Wasser oder verdünnte Salzsäure, das Lösemittel sowie der Radikalstarter zugeführt wurden. Ein Eintauchrohr zur kontinuierlichen Zuführung von verflüssigtem Chlor, das vorteilhaft in das Reaktionsgemisch eintaucht und nach Möglichkeit unterhalb des Rührerniveaus endet, war vorhanden. Die Wasser-oder Salzsäuremenge wurde in jedem Versuchsfall so bemessen, daß während der Chlorierung eine 29 % (m/m) bis 35 % (m/m)ige Salzsäure entstand, so daß ein Abführen des bei der Chlorierung entstehenden Chlorwasserstoffs nicht nötig wurde.

Durch Druck- und Temperaturmeßeinrichtungen wurde die Chloriereinheit sicherheitstechnisch abgesichert.

### Vergleichsbeispiel A

Eine Mischung aus 730 kg Paraffinkohlenwasserstoffen mit 17 bis 26 Kohlenstoffatomen im Molekül, 1 600 kg Tetrachlorkohlenstoff (40 % (m/m) bezogen auf das hergestellte Chlorparaffin) sowie 3 900 kg Wasser wurden in Gegenwart von 0,3 % Dilauroylperoxid, bezogen auf das hergestellte Chlorparaffin, bei einer Temperatur von 84 bis 103 °C und einem Druck, der im Verlaufe der Chlorierung bis auf 5,3 bar gesteigert wurde, mit 3 570 kg flüssigem Chlor umgesetzt. Die gewünschte Chlormenge konnte in einem Zeitraum von ca. 5 Stunden eingebracht werden, wobei das eingetragene Chlor vollständig, das heißt zu mehr als 99,9 % (m/m) abreagiert war. Nach beendeter Chlorierung wurde der Rührer abgestellt, um Phasentrennung innerhalb von 10 Minuten zu erreichen. Die organische Phase wurde zur Entfernung des Lösemittels abgelassen.

Das Chlorparaffin hatte einen Chlorgehalt von 72 % (m/m), die Ausbeute betrug 2 500 kg festen Chlorparaffins.

### Vergleichsbeispiel B

Die Chlorierung wurde in ähnlicher Weise wie im Vergleichsbeispiel A beschrieben durchgeführt mit der Abwandlung, daß an Stelle von Tetrachlorkohlenstoff Chloroform eingesetzt wurde und die zugeführte Chlormenge um den Anteil, der benötigt wird, um das eingesetzte Chloroform zu Tetrachlorkohlenstoff zu chlorieren, erhöht wurde. Das erhaltene Chlorparaffin hatte einen Chlorgehalt von ca. 72 % (m/m); die Phasentrennung wurde innerhalb von 10 Minuten bei der Chlorierung von 84 bis 102 °C erreicht.

### Vergleichsbeispiel C

In Abänderung der Versuchsbedingungen wurden bei ansonst konstant Einsatzmengen die Mengen an Tetrachlorkohlenstoff als Lösemittel auf 1 400 kg entsprechend 36 % (m/m), bezogen auf das hergestellte Chlorparaffin, reduziert. Bei einer Chlorierzeit von ca. 5,5 h und einer Phasentrennzeit von ca. 10 Minuten bei einer Temperatur von 84 bis 102 °C wurden 2 500 kg Chlorparaffin mit einem Chlorgehalt von 72 % (m/m) hergestellt. Die Ablaufzeit in die nachgeschaltete Apparatur zur Entfernung des Lösemittels betrug 10 Minuten.

### Vergleichsbeispiel D

In Abänderung der Versuchsbedingungen wurden bei ansonst konstanten Einsatzmengen die Mengen an Tetrachlorkohlenstoff als Lösemittel auf 900 kg entsprechend 26 % (m/m), bezogen auf das hergestellte Chlorparaffin, reduziert. Bei einer Chlorierzeit von ca. 5,5 h und einer Phasentrennzeit von 20 Minuten bei einer Temperatur von 95 °C wurden 2 500 kg Chlorparaffin mit einem Chlorgehalt von ca. 72 % (m/m) hergestellt. Zwischen den Phasen wurde eine Mulmschicht beobachtet. Das Ablaufen des Produktes in die nachgeschaltete Apparatur dauerte 12 Minuten und war nur geringfügig behindert.

### Vergleichsbeispiel E

In Abänderung der Versuchsbedingungen wurden bei ansonst konstanten Einsatzmengen die Mengen an Tetrachlorkohlenstoff als Lösemittel auf 600 kg entsprechend 19 % (m/m), bezogen auf das hergestellte Chlorparaffin, reduziert. Bei einer Chlorierzeit von ca. 5,5 h und einer Phasentrennzeit von 25 Minuten bei einer Temperatur von 94 °C wurden 2 500 kg Chlorparaffin mit einem Chlorgehalt von ca. 72 % (m/m) hergestellt. Zwischen den Phasen wurde eine Mulmschicht festgestellt, die nach der Menge her deutlich höher als im Versuchsbeispiel D war. Die Ablaufzeit in die nachgeschaltete Apparatur zur Entfernung des Lösemittels erhöhte sich auf 23 Minuten.

### Vergleichsbeispiel F

In einer weiteren Abänderung der Versuchsbedingungen wurden bei ansonst konstanten Einsatzmengen die Mengen an Tetrachlormethan auf 300 kg entsprechend 11 % (m/m), bezogen auf das hergestellte Chlorparaffin, reduziert. Bei einer Chlorierzeit von ca. 7 Stunden und einer Phasentrennung von ca. 40 Minuten bei einer Temperatur von 94 °C wurden 2 500 kg Chlorparaffin mit einem Chlorgehalt von ca. 72 % (m/m) hergestellt. Die Ablaufzeit in die nachgeschaltete Apparatur zur Entfernung des Lösemittels erhöhte sich auf ca. 60 Minuten. Aufgrund einer starken Mulmschicht zwischen den beiden Phasen erhöhte sich die Abdestillierzeit des Lösemittels infolge mitgerissener Salzsäure erheblich.

### Beispiel 1

In einer weiteren Abänderung der Versuchsbedingungen wurde bei ansonsten konstanten Einsatzmengen auf das Lösemittel Tetrachlorkohlenstoff oder Chloroform ganz verzichtet. Bei einer Chlorierzeit von ca. 7,5 h und einer Phasentrennzeit von ca. 30 Minuten, die aufgrund des fehlenden Lösemittels bei einer Temperatur von 135 °C durchgeführt werden konnte, wurden wieder 2 500 kg Chlorparaffin hergestellt. Die Phasentrennung war sehr gut ausgebildet, ohne Anzeichen einer Mulmschicht. Die Salzsäurephase war ihrerseits sehr sauber und frei von Chlorparaffinflocken. Die Ablaufzeit in das nachgeschaltete Aggregat betrug 55 Minuten. Aufgrund der guten Phasentrennung wurden Restmengen an Salzsäure aus der Chlorparaffin-Phase rasch und vollständig entfernt.

Um eine auch ohne Lösemittel akzeptable Chlorierzeit zu erreichen, muß der Abstand des Impellerrührers zum Rührwerksboden so gering sein, und der Durchmesser des Rührers so bemessen sein, daß das angesaugte Chlorparaffin/Paraffingemisch ausreichend gut zerteilt und mit der wäßrigen Phase und dem vorhandenen Chlor innig vermischt wird. Die Reaktion des Chlors mit dem Chlorparaffin/Paraffingemisch war dann gegeben. Nach erfolgter Umsetzung wurde der Ansatz auf eine Temperatur bis zu 140 °C aufgeheizt, um eine einwandfreie Trennung der Phasen zu erreichen.

### Beispiel 2

In weiteren Versuchen wurde bei gleichen Versuchsbedingungen und konstanten Einsatzmengen in Analogie zum Beispiel 1 der eingesetzte Paraffinkohlenwasserstoff hinsichtlich Kettenlängenverteilung variiert. In jedem Fall wurde nach beendeter Chlorierung bei Temperaturen bis 140 °C Phasentrennung erreicht, ohne daß eine die weitere Aufarbeitung störende Mulmschicht beobachtet wurde.

## Claims

1. A process for preparing a C₁₄-C₄₀ chlorinated paraffin having a chlorine content of more than 60% by weight from a C₁₄-C₄₀ paraffin reactant being a saturated or unsaturated hydrocarbon which can already contain chlorine atoms, said process comprising:
reacting the C₁₄-C₄₀ paraffin reactant with an amount of liquid chlorine which reacts completely with the paraffin to form a chlorinated paraffin phase of said C₁₄-C₄₀ chlorinated paraffin having a chlorine content of more than 60% by weight, under a pressure of up to 10 bar in the absence of an organic solvent at an elevated temperature ranging from 75°C up to 140°C, in the presence of a free radical source, said C₁₄-C₄₀ paraffin being in intimate mixture with an aqueous phase, the intimate mixture being maintained by stirring, said reacting being continued until a chlorinated paraffin phase and an aqueous hydrochloric acid phase have been formed, and
separating said chlorinated paraffin phase from said aqueous phase, wherein said separating step is carried out by cessation of said stirring.

2. The process according to claim 1, wherein said C₁₄-C₄₀ paraffin reactant is saturated.

3. The process according to claim 1 or claim 2, wherein said C₁₄-C₄₀ paraffin reactant has been prechlorinated.

4. The process according to any of claims 1 to 3, there being used as aqueous phase dilute hydrochloric acid.

5. The process according to any of claims 1 to 4, wherein said stirring is carried out with an impeller stirrer.

6. The process according to any of claims 1 to 5, wherein said pressure ranges from 2 to 6 bar.

7. The process according to any of claims 1 to 6, wherein said aqueous phase is present in an amount sufficient to absorb the hydrogen chloride formed during the reaction, or to form a dilute hydrochloric acid having a strength of from 29% (m/m) to 35% (m/m).

8. A process for preparing highly chlorinated paraffins having a chlorine content of more than 60% by weight at a temperature of from 75 to 140°C and a pressure of up to 10 bar by reacting a C₁₄-C₄₀ paraffin being a saturated or unsaturated hydrocarbon which can already contain chlorine atoms with liquid chlorine, wherein
the chlorine reacts completely, the reaction is carried out in the presence of an amount of water such that during chlorination there is formed hydrochloric acid having a strength of from 29% (m/m) to 35% (m/m), in the presence of a free-radical source and in the absence of an organic solvent.

## Patentansprüche

1. Verfahren zur Herstellung eines chlorierten C₁₄-C₄₀ Paraffins mit einem Chlorgehalt von mehr als 60 Gew.-% aus einem C₁₄-C₄₀ Paraffinreaktanten, der ein gesättigter oder ungesättigter Kohlenwasserstoff ist, welcher bereits Chloratome enhalten kann, wobei das Verfahren umfaßt:
Umsetzen des C₁₄-C₄₀ Paraffinreaktanten mit einer Menge an flüssigem Chlor, die vollständig mit dem Paraffin reagiert und eine chlorierte Paraffinphase des chlorierten C₁₄-C₄₀ Paraffins mit einem Chlorgehalt von mehr als 60 Gew.-% bildet, unter einem Druck von bis zu 10 bar in Abwesenheit eines organischen Lösungsmittels bei einer erhöhten Temperatur im Bereich von 75 °C bis zu 140 °C in Gegenwart einer Quelle für freie Radikale, wobei das C₁₄-C₄₀ Paraffin im innigen Gemisch mit einer wäßrigen Phase vorliegt, das innige Gemisch durch Rühren aufrechterhalten wird und die Reaktion fortgesetzt wird, bis eine chlorierte Paraffinphase und eine wäßrige Salzsäurephase gebildet worden ist, und
Trennen der chlorierten Paraffinphase von der wäßrigen Phase, worin der Trennschritt dadurch durchgeführt wird, daß das Rühren beendet wird.

2. Verfahren nach Anspruch 1, worin der C₁₄-C₄₀ Paraffinreaktant gesättigt ist.

3. Verfahren nach Anspruch 1 oder 2, worin der C₁₄-C₄₀ Paraffinreaktant vorher anchloriert worden ist.

4. Verfahren nach einem der Ansprüche 1 bis 3, wobei als wäßrige Phase verdünnte Salzsäure verwendet wird.

5. Verfahren nach einem der Ansprüche 1 bis 4, worin das Rühren mit einem Impeller-Rührer durchgeführt wird.

6. Verfahren nach einem der Ansprüche 1 bis 5, worin der Druck im Bereich von 2 bis 6 bar liegt.

7. Verfahren nach einem der Ansprüche 1 bis 6, worin die wäßrige Phase in einer dazu ausreichenden Menge vorliegt, daß der während der Reaktion gebildete Chlorwasserstoff absorbiert wird oder eine verdünnte Salzsäure mit einer Stärke von 29 % (m/m) bis 35 % (m/m) gebildet wird.

8. Verfahren zur Herstellung von hochchlorierten Paraffinen mit einem Chlorgehalt von mehr als 60 Gew.-% bei einer Temperatur von 75 bis 140 °C und einem Druck von bis zu 10 bar durch Umsetzen eines C₁₄-C₄₀ Paraffins, das ein gesättigter oder ungesättigter Kohlenwasserstoff ist, welcher bereits Chloratome enhalten kann, mit flüssigem Chlor, worin
das Chlor vollständig reagiert, die Reaktion in Gegenwart einer derartigen Wassermenge durchgeführt wird, daß während der Chlorierung Salzsäure mit einer Stärke von 29 % (m/m) bis 35 % (m/m) gebildet wird, in Gegenwart einer Quelle für freie Radikale und in Abwesenheit eines organischen Lösungsmittels.

## Revendications

1. Procédé de préparation d'une paraffine en C₁₄-C₄₀ chlorée ayant une teneur en chlore supérieure à 60 % en poids à partir d'un réactif consistant en une paraffine en C₁₄-C₄₀ qui est un hydrocarbure saturé ou insaturé, lequel peut éventuellement déjà contenir des atomes de chlore, le procédé comprenant :
la réaction du réactif consistant en une paraffine en C₁₄-C₄₀ avec une quantité de chlore liquide, qui réagit totalement avec la paraffine et forme une phase de paraffine chlorée comprenant la paraffine en C₁₄-C₄₀ chlorée ayant une teneur en chlore supérieure à 60 % en poids, sous une pression allant jusqu'à 10 bars, en l'absence de solvant organique, à une température élevée dans la gamme de 75° à 140°C, en présence d'une source de radicaux libres, la paraffine en C₁₄-C₄₀ se présentant en mélange intime avec une phase aqueuse, le mélange intime étant maintenu par agitation et la réaction étant menée jusqu'à ce qu'une phase paraffinique chlorée et une phase d'acide chlorhydrique aqueuse se forme ; et
la séparation de la phase paraffinique chlorée de la phase aqueuse, l'étape de séparation étant conduite de sorte que l'agitation soit stoppée.

2. Procédé selon la revendication 1, dans lequel le réactif à base de paraffine en C₁₄-C₄₀ est saturé.

3. Procédé selon la revendication 1 ou 2, dans lequel le réactif à base de paraffine en C₁₄-C₄₀ est préalablement chloré.

4. Procédé selon l'une des revendications 1 à 3, dans lequel on utilise en tant que phase aqueuse de l'acide chlorhydrique dilué.

5. Procédé selon l'une des revendications 1 à 4, dans lequel l'agitation est conduite au moyen d'un agitateur à pâles.

6. Procédé selon l'une des revendications 1 à 5, dans lequel la pression est comprise entre 2 et 6 bars.

7. Procédé selon l'une des revendications 1 à 6, dans lequel la phase aqueuse est présente en une quantité suffisante pour que le chlorure d'hydrogène formé pendant la réaction soit absorbé ou que de l'acide chlorhydrique dilué ayant une force de 29 % m/m à 35 % m/m soit formé.

8. Procédé de préparation d'une paraffine hautement chlorée ayant une teneur en chlore supérieure à 60 % en poids, à une température de 75 à 140°C et une pression allant jusqu'à 10 bars, par réaction d'une paraffine en C₁₄-C₄₀ consistant en un hydrocarbure saturé ou insaturé pouvant déjà contenir des atomes de chlore, avec du chlore liquide, dans lequel le chlore réagit en totalité, la réaction est menée en présence d'une quantité d'eau telle que, pendant la chloration, il se forme de l'acide chlorhydrique ayant une force de 29 % m/m à 35 % m/m, en présence d'une source de radicaux libres et en l'absence d'un solvant organique.
